# EUROPEAN PATENT APPLICATION

(11) **EP 4 566 598 A1**
(43) Date of publication of application: **11.06.2025**
(21) Application number: 24184796.1
(22) Date of filing: 26.06.2024
(51) Int. Cl.: A61K 31/22, A61K 31/70, A61K 31/7004, A61P 9/00, A61P 9/04

(54) **COMPOSITIONS PROVIDING SGLT2I AND BHB ACTIVITY AND METHODS OF USE TO TREAT HUMAN DISEASE**

(30) Priority: 04.12.2023 US 202318528462
(71) Applicant: ZoSaLa Pharma, Inc, Atlanta, GA 30307 (US); Georgia State University Research Foundation, Inc., Atlanta, GA 30303 (US)
(72) Inventor: LA TERZA, Vincent, Atlanta, GA 30307 (US); HAN, Young-Min, Buford, GA 30519 (US); RAUTIO, Jarkko, Kuopio 70200 (FI)
(74) Representative: Schlich

(57) **Abstract**

Compositions of compounds with SGLT2i activity in combination with compounds having BHB activity. These can be used in methods of treatment for a variety of human diseases such as cognitive deficits, Alzheimer's, kidney disease, heart failure and aortic aneurysms and dissection. The methods allow therapeutic levels of both SGLT2i and BHB activity to be achieved and maintained for as many hours per day as deemed optimal for the patient in need of therapy.

## Description

### BACKGROUND

Substantial evidence from studies in animals and persons suggests that the metabolite BHB offers health benefits including improving the performance of the heart and brain and is associated with protecting cells from age related damage. Most healthy persons eating a western diet will have circulating BHB in a concentration of ~.15mM. A concentration >0.6mM is considered high. All publications mentioned or cited herein are incorporated herein by reference to disclose and describe the methods and/or materials described within such publications. Maintaining BHB in a range of ~1.0mM-2.0mM sustained for 8-10 hours per day is deemed safe and probably needed to reap the benefits that BHB may offer (Shingo Takahara et al. - "Chronic Exogenous Ketone Supplementation Blunts the Decline of Cardiac Function in the Failing Heart," ESC Heart Failure 8, no. 6 (October 6, 2021): 5606-12). The ketogenic diet and associated maintenance of circulating ketone bodies is of interest for the treatment of a variety of disorders and also as a potential means to delay or combat deleterious processes associated with aging. The primary ketones made by the human body, mostly in the liver, are acetoacetate (AcAc) and beta-hydroxybutyrate (BHB). BHB is a chiral molecule and occurs as two enantiomers (R/D-BHB) and (S/L-BHB). R-beta hydroxybutyrate (R-BHB) is the predominant product of mammalian ketogenesis. The R(D)-enantiomer of BHB is a metabolic intermediate and serves in generating cellular energy. Both BHB enantiomers serve in signaling and in modulating epigenetic activity. S/L BHB is far more stable in human plasma than R/D BHB (Brianna J. Stubbs et al. "On the Metabolism of Exogenous Ketones in Humans," Frontiers in Physiology 8 (2017): 848). There is emerging evidence that the chiral nature of BHB may affect its signaling and epigenetic activity (Xingrun Zhang et al., "Molecular Basis for Hierarchical Histone De-β-Hydroxybutyrylation by SIRT3," Cell Discovery 5 (2019): 3).

Fat mobilized from adipose tissue, such as during fasting, may be transported to the liver and converted into BHB (almost all R-BHB). Once absorbed into a cell, R-BHB may be broken down to generate Acetyl-CoA that is further metabolized into ATP. R-BHB, once converted into acetyl-CoA, also contributes to signaling indirectly or directly.

The metabolism of R-BHB and the myriad biological activities of R-BHB are well documented and described in the scientific literature ((Judith Campisi et al., "From Discoveries in Ageing Research to Therapeutics for Healthy Ageing, Nature 571, no. 7764 (July 2019): 183-92), (John C. Newman and Eric Verdin, "β-Hydroxybutyrate: A Signaling Metabolite, Annual Review of Nutrition 37 (August 21, 2017): 51-76), (Benjamin T. Bikman and Kelsey H. Fisher-Wellman, "The Metabolic Effects of Ketones, International Journal of Molecular Sciences 22, no. 15 (August 2, 2021): 8292), (Hubert Kolb et al. "Ketone Bodies: From Enemy to Friend and Guardian Angel, BMC Medicine 19, no. 1 (December 9, 2021): 313), (Dabke and Anibh M. Das, "Mechanism of Action of Ketogenic Diet Treatment: Impact of Decanoic Acid and Beta-Hydroxybutyrate on Sirtuins and Energy Metabolism in Hippocampal Murine Neurons, Nutrients 12, no. 8 (August 8, 2020): E2379), (Souad Nasser et al., "Effects of Ketogenic Diet and Ketone Bodies on the Cardiovascular System: Concentration Matters, World Journal of Diabetes 11, no. 12 (December 15, 2020): 584-95), (Sirven et al. - The Ketogenic Diet for Intractable Epilepsy in Adults: Preliminary Results," Epilepsia 40, no. 12 (December 1999): 1721-26), (Brianna J. Stubbs et al., "From Bedside to Battlefield: Intersection of Ketone Body Mechanisms in Geroscience with Military Resilience, GeroScience 43, no. 3 (June 2021): 1071-81), (Latha Nagamani Dilliraj et al., The Evolution of Ketosis: Potential Impact on Clinical Conditions," Nutrients 14, no. 17 (September 1, 2022): 3613)). The metabolism and function of S-BHB is less well understood. S-BHB is a common intracellular intermediate of the fatty acid beta-oxidation pathway but is not usually found in the circulation beyond trace concentrations and cannot be oxidized by the same pathway as R-BHB (Webber RJ, Edmond J., "Utilization of L(+)-3-hydroxybutyrate,D(-)-3-hydroxybutyrate, acetoacetate, and glucose for respiration and lipid-synthesis in 18-day-old rat. J Biol Chem.1977;252(15):5222-6. doi:10.1016/S0021-9258(19)63335-1).

### ELEVATED BHB IS BENEFICIAL TO TREAT AORTIC DISEASE

Aortic aneurysm is a progressive focal dilation of the aorta characterized by altered cellular and matrix organization. The infrarenal abdominal aorta (AAA) is the most common site of aortic aneurysm formation, followed by the thoracic aorta (TAA) (Reed WD, Ozand PT. Enzymes of L-(+)-3-hydroxybutyrate metabolism in the rat. Arch Biochem Biophys. 1980;205(1):94-103.doi:10.1016/0003-9861(80)90087-9), (Raymundo Alain Quintana and W. Robert Taylor, "Cellular Mechanisms of Aortic Aneurysm Formation," Circulation Research 124, no. 4 (February 15, 2019): 607-18, https://doi.org/10.1161/CIRCRESAHA.118.313187). The altered aortic wall structure and geometry of an aneurysmal dilation predisposes the wall to failure when constantly subjected to cardiac cycle pulsatile perfusion stress. Aortic *dissection* is a luminal disruption of the aortic wall that does not penetrate the outer media and adventitia, causes delamination of the media of the aorta, and predisposes it to aneurysmal dilation and rupture (V. Tchana-Sato, N. Sakalihasan, and J. O. Defraigne, "[Aortic dissection]," Revue Medicale De Liege 73, no. 5-6 (May 2018): 290-95). Aortic dissection is often classified as either heritable or sporadic and most often involves the thoracic aortic (TAAD) (Sherene Shalhub et al., "Characterization of Syndromic, Nonsyndromic Familial, and Sporadic Type B Aortic Dissection," Journal of Vascular Surgery 73, no. 6 (June 2021): 1906-1914.e2). The prevalence of abdominal aortic aneurysm (AAA) is 9% in patients older than 65 years of age, and the incidence of thoracic aortic aneurysm (TAA), which may be precede or follow dissection (TAAD), is estimated to be about 5 patients per 100,000 persons-years (R. Scott McClure et al., "Epidemiology and Management of Thoracic Aortic Dissections and Thoracic Aortic Aneurysms in Ontario, Canada: A Population-Based Study," The Journal of Thoracic and Cardiovascular Surgery 155, no. 6 (June 2018): 2254-2264.e4). The first indication of aortic disease is often fatal dissection or rupture, which in the case of TAAD, may strike relatively young, otherwise vibrant, healthy people. Aortic rupture or dissection accounts for up to 16,000 deaths annually in the US (Halah Abdulameer et al., "Epidemiology of Fatal Ruptured Aortic Aneurysms in the United States (1999-2016)," Journal of Vascular Surgery 69, no. 2 (February 2019): 378-384.e2,). No medications have been clinically shown to effectively prevent aortic disease progression.

### BENEFICIAL EFFECTS OF ELEVATED BHB IN RODENT MODELS OF AAA

Ming Zou, Ph.D, MD and Young-Min Han, Ph.D discovered that hydroxybutyrate or BHB, when elevated, impedes AAA induction and remarkably, reverses established AAA in two animal models. This finding was independently replicated by investigators at Washington University in a third model of AAA (Sastriques-Dunlop S et al., "Ketosis Prevents Abdominal Aortic Aneurysm Rupture Through CCR2 Downregulation and Enhanced MMP Balance," bioRxiv: The Preprint Server for Biology, February 22, 2023.) It is well recognized that BHB has pleiotropic activities three of which are: i) as a fuel molecule; ii) as a signaling molecule and iii) as an epigenetic regulator. The Zou lab determined that both enantiomers of BHB have similar efficacy in deterring and healing AAA. S-BHB efficacy strongly suggests that BHB signaling and epigenetic activities confer primary benefit in AAA, because S-BHB is not a fuel molecule.

### BHB ANTI-INFLAMMATORY SIGNALING ACTIVITY

Although blunting inflammation alone has not provided efficacy in human trials of AAA, inflammation certainly contributes to AAA progression (Zhen Yuan et al., "Abdominal Aortic Aneurysm: Roles of Inflammatory Cells," Frontiers in Immunology 11 (February 3, 2021): 609161,). Inflammasomes are intracellular molecular protein scaffolds that can induce pyroptosis (an inflammatory form of cell death) and necroptosis (a lytic form of inflammatory cell death) by cleaving the N-terminal of pro-IL-1b and pro-IL- 18 with caspase-1 (Vijay A.K. Rathinam and Francis Ka-Ming Chan, "Inflammasome, Inflammation and Tissue Homeostasis," Trends in Molecular Medicine 24, no. 3 (March 2018): 304-18,). Inhibiting or silencing the NLRP3-inflammasome or the STING inflammasome has been shown to limit AAA progression ((Pingping Ren et al., "Targeting the NLRP3 Inflammasome With Inhibitor MCC950 Prevents Aortic Aneurysms and Dissections in Mice," Journal of the American Heart Association: Cardiovascular and Cerebrovascular Disease 9, no. 7 (March 30, 2020): e014044,), (Wei Luo et al., "Critical Role of Cytosolic DNA and Its Sensing Adaptor STING in Aortic Degeneration, Dissection, and Rupture," Circulation 141, no. 1 (January 7, 2020): 42-66)). BHB inhibits the NLRP3inflammasome and the STING inflammasome (Yun-Hee Youm et al., "The Ketone Metabolite β-Hydroxybutyrate Blocks NLRP3 Inflammasome-Mediated Inflammatory Disease," Nature Medicine 21, no. 3 (March 2015): 263-69), (Shilu Luo et al., "βHydroxybutyrate against Cisplatin-Induced Acute Kidney Injury via Inhibiting NLRP3 Inflammasome and Oxidative Stress," International Immunopharmacology 111 (October 2022): 109101). The Sayed lab found that a ketogenic diet or the administration of exogenous ketones decreased CCR2 receptor expression and muted inflammatory pathways to impede the induction of AAA and arrest AAA expansion in the PPE BAPN rat model of aneurysm and dissection (S et al., "Ketosis Prevents Abdominal Aortic Aneurysm Rupture Through CCR2 Downregulation and Enhanced MMP Balance").

### BHB EPIGENETIC ACTIVITY AND CELLULAR RENEWAL

Epigenetics describes the molecular basis for heritable changes in gene expression that do not involve nucleotide sequence changes. Much has been learned about highly dynamic, focal changes in chromatin structure, mediated by the activities of histone post-translational modifications (HPTMs), DNA methylation, and non-coding RNAs (Yanqiu He et al., "β-Hydroxybutyrate as an Epigenetic Modifier: Underlying Mechanisms and Implications," Heliyon 9, no. 11 (November 2023): e21098,). It is well established that BHB induces anti-oxidative, anti-inflammatory, and cardioprotective features via binding to several target proteins, including a key class of epigenetic enzymes, histone deacetylases (HDACs), or G protein-coupled receptors (GPCRs) (A. Kyoung Lee et al., "β-Hydroxybutyrate Suppresses Lipid Accumulation in Aged Liver through GPR109A-Mediated Signaling," Aging and Disease 11, no. 4 (July 2020): 777-90), (Xinhui Li et al., "βHydroxybutyrate Ameliorates Aβ-Induced Downregulation of TrkA Expression by Inhibiting HDAC1/3 in SH-SY5Y Cells," American Journal of Alzheimer's Disease and Other Dementias 35 (2020): 1533317519883496), (Newman and Verdin, "β-Hydroxybutyrate," August 21, 2017). In recent years it has become increasingly clear that the plasma level of BHB regulates a series of epigenetic phenomena, including the addition or removal of chromatin markers, such as Kme, Kac, and Kbhb. By altering the expression of panels of genes, these activities impart phenotypic changes and modify cell function. The beneficial role of BHB in cardiometabolic disease has been reviewed (Nadia Bendridi et al., "Ketone Bodies as Metabolites and Signalling Molecules at the Crossroad between Inflammation and Epigenetic Control of Cardiometabolic Disorders," International Journal of Molecular Sciences 23, no. 23 (November 23, 2022)). Detailed studies of the phenotypic shift that drives AAA in animal models as observed by the Zou lab and recent published reports from other leading labs investigating aortic disease, reveal that epigenetic modulation is salient in transitions from healthy contractile vSMC to pathological synthetic phenotype (Abhijit Chakraborty et al., "Epigenetic Induction of Smooth Muscle Cell Phenotypic Alterations in Aortic Aneurysms and Dissections," Circulation 148, no. 12 (September 19, 2023): 959-77,), (Frank M. Davis et al., "Inhibition of Macrophage Histone Demethylase JMJD3 Protects against Abdominal Aortic Aneurysms," The Journal of Experimental Medicine 218, no. 6 (June 7, 2021): e20201839,).

### BENEFICIAL EFFECTS OF SGLT2 INHIBITORS IN RODENT MODELS OF AAA

Dapagliflozin (Farxiga) was FDA approved for type 2 diabetes in 2014 and obtained additional approval to treat heart failure (2020) and kidney disease (2021) (https://www.drugs.com/history/farxiga.html). In an animal model of AAA induced by porcine pancreatic protease (PPE), dapagliflozin significantly impeded aneurysmal induction and expansion (Haole Liu et al., "Dapagliflozin Ameliorates the Formation and Progression of Experimental Abdominal Aortic Aneurysms by Reducing Aortic Inflammation in Mice," Oxidative Medicine and Cellular Longevity 2022 (2022): 8502059). Protection may have been due to the significant reduction in aortic accumulation of macrophages, CD4+ T cells, and B cells. The investigators noted that SGLT2i preservation of autophagy in the context of cellular stress may have contributed to the significant attenuation of medial vSMC loss. The SGLT2 inhibitor empagliflozin impedes AAA in the apoE-/- Ang II induction model (Rebeca Ortega et al., "SGLT-2 (Sodium-Glucose Cotransporter 2) Inhibition Reduces Ang II (Angiotensin II)-Induced Dissecting Abdominal Aortic Aneurysm in ApoE (Apolipoprotein E) Knockout Mice," Arteriosclerosis, Thrombosis, and Vascular Biology 39, no. 8 (August 2019): 1614-28). Efficacy in the model was attributed to reduced expression of chemokines (CCL-2 [chemokine (C-C motif) ligand 2] and CCL-5 [chemokine (CC motif) ligand 5]), VEGF (vascular endothelial growth factor), and MMP (matrix metalloproteinase)-2 and MMP-9 in suprarenal aortic walls, in comparison to mice subject to induction but not treated with empagliflozin. Like BHB, SGLT2 inhibitors exhibit pleiotropic activities. A very recent review (April 2023) by Jin et al. enumerates several ways in which SGLT2i benefits various cells integral to aortic wall health and function (24 Zhongtiao Jin et al., "Perspective of SGLT2i in the Treatment of Abdominal Aortic Aneurysms," Journal of Cardiovascular Pharmacology 81, no. 4 (April 1, 2023): 241-47). Clinical studies have shown that AAA is highly correlated with endothelial dysfunction (25 DeRoo E, Stranz A, Yang H, et al. Endothelial dysfunction in the pathogenesis of abdominal aortic aneurysm. Biomolecules. 2022;12:509). SGLT2i favorably regulates the proliferation, migration, differentiation, survival, and senescence of endothelial cells (ECs) (26 Durante W, Behnammanesh G, Peyton KJ. Effects of sodium-glucose Co-transporter 2 inhibitors on vascular cell function and arterial remodeling. Int J Mol Sci. 2021;22:8786). SGLT2i has potent antioxidant and anti-inflammatory effects on ECs, and its direct inhibition of endothelial cell NHE1 attenuates endothelial inflammation (Uthman L, Li X, Baartscheer A, et al. Empagliflozin reduces oxidative stress through inhibition of the novel inflammation/NHE/[Na(+)](c)/ ROS-pathway in human endothelial cells. Biomed Pharmacother. 2022;146:112515). SGLT2i deters vSCM phenotypic shift. Empagliflozin inhibits the secretion of IL-1b and IL-18 dependent on TRAF3IP2/ROS/NLRP3/caspase-1 in human VSMCs, weakens the inflammatory signal transduction and oxidative stress induced by IL-17a, and inhibits the phenotypic transformation of VSMCs (Sukhanov S, Higashi Y, Yoshida T, et al. The SGLT2 inhibitor Empagliflozin attenuates interleukin-17A-induced human aortic smooth muscle cell proliferation and migration by targeting TRAF3IP2/ROS/ NLRP3/Caspase-1-dependent IL-1b and IL-18 secretion. Cell Signal. 2021;77:109825). Canagliflozin stimulates the expression of heme oxygenase-1 in vSMCs by activating the ROS- Nrf2 (NF-E2-related factor-2) pathway, leading to antiproliferative and antimigratory effects (Behnammanesh G, Durante GL, Khanna YP, et al. Canagliflozin inhibits vascular smooth muscle cell proliferation and migration: role of heme oxygenase-1. Redox Biol. 2020;32:101527). In an in vitro experiment using rat aortic smooth muscle cells, empagliflozin significantly decreased aortic smooth muscle cell proliferation in a dose dependent manner (Takahashi H, Nomiyama T, Terawaki Y, et al. Combined treatment with DPP-4 inhibitor linagliptin and SGLT2 inhibitor empagliflozin attenuates neointima formation after vascular injury in diabetic mice. Biochem Biophys Rep. 2019; 18: 1 00640). Perivascular adipose tissue (PVAT) inhabits tissue adjacent to the adventitial layer of the aortic wall. There is an excessive accumulation of PVAT in the wall of ruptured AAA (Ye T, Zhang G, Liu H, et al. Relationships between perivascular adipose tissue and abdominal aortic aneurysms. Front Endocrinol. 2021;12: 704845). When the volume of PVAT increases, the expression of inflammatory cells, cytokines, and chemokines increases, resulting in vascular injury (Chen Y, Qin Z, Wang Y, et al. Role of inflammation in vascular disease- related perivascular adipose tissue dysfunction. Front Endocrinol (Lausanne). 2021;12:710842). Luseogliflozin can inhibit PVAT remodeling by suppressing platelet-derived growth factor-B gene expression and reducing macrophage infiltration, thereby reducing intimal hyperplasia after bilateral femoral artery wire injury in high-fat diet fed mice (33 Mori Y, Terasaki M, Hiromura M, et al. Luseogliflozin attenuates neo- intimal hyperplasia after wire injury in high-fat dietfed mice via inhibition of perivascular adipose tissue remodeling. Cardiovasc Diabetol. 2019;18:143). Ipragliflozin attenuated inflammation, fibrosis, and adipocyte death of abdominal PVAT in mice fed with Western diet (Mori K, Tsuchiya K, Nakamura S, et al. Ipragliflozin-induced adipose expansion inhibits cuff-induced vascular remodeling in mice. Cardiovasc Diabetol. 2019;18:83). Yet another favorably activity of SGLT2i is the ability to reduce blood pressure by lowering plasma Na+ and inhibiting K+ channels to promote vasodilatation (Kobeissi E, Hibino M, Pan H, Aune D. Blood pressure, hypertension, and the risk of abdominal aortic aneurysms: a systematic review and meta- analysis of cohort studies. Eur J Epidemiol. 2019;34:547-555). The accumulated experience with SGLT2i in millions of treated patients and considerable understanding of the myriad molecular pathways this class of drugs engages that promises with favorable outcomes for AAA patients, offers a very strong rationale for moving forward to evaluate dapagliflozin in the pilot trial proposed by ZoSala in this project.

Vascular smooth muscle cells (vSMC) have the remarkable ability to acquire different phenotypes, including synthetic, fibroblast, and macrophage-like phenotypes. These vSMC transformations occur in the aortic wall of AAA patients (Ploingarm Petsophonsakul et al., "Role of Vascular Smooth Muscle Cell Phenotypic Switching and Calcification in Aortic Aneurysm Formation," Arteriosclerosis, Thrombosis, and Vascular Biology 39, no. 7 (July 2019): 1351-68). A growing body of evidence links pathological and beneficial changes in VSCMs to mechanisms that regulate the coordinated activity of a plurality of gene products. In line with the idea that modulation of a plurality of gene products steers phenotypic switching of VSMCs is a body of work underscoring the role of epigenetics in AAA (Rijan Gurung et al., "Genetic and Epigenetic Mechanisms Underlying Vascular Smooth Muscle Cell Phenotypic Modulation in Abdominal Aortic Aneurysm," International Journal of Molecular Sciences 21, no. 17 (August 31, 2020): E6334). Histone deacetylases (HDACs) have been shown to modulate the expression of VSMC genes involved in AAA and regulate differentiation, contractility, proliferation, inflammation, and ECM deposition. Three HDAC inhibitors, MS-275, MC-1569 and MCT-1 have been shown to reduce AAA in murine models (Gurung et al. Douwe Pons et al., "Epigenetic Histone Acetylation Modifiers in Vascular Remodeling: New Targets for Therapy in Cardiovascular Disease," European Heart Journal 30, no. 3 (February 2009): 266-77). A large study that assessed peripheral blood mononuclear cell DNA from AAA patients and controls reported that global DNA methylation was significantly higher in men with large AAA compared to small AAA.

### REDUCING INFLAMMATION IS BENEFICIAL IN AORTIC DISEASE

A plethora of clinical and preclinical data describe a crucial role for inflammation in aortic disease. Many studies have identified immune infiltrate within the middle and outer tunics of dissected aortic specimens from patients with aortic diseases. It has also been observed that the recall and activation of macrophages inside the middle tunic are key events in the early phases of aneurysmal development. Macrophages are able to release metalloproteinases (MMPs) and pro-inflammatory cytokines which, in turn, give rise to matrix degradation and neoangiogenesis. An imbalance between the production of MMPs and MMP tissue inhibitors is thought to be pivotal in the extracellular matrix degradation underlying aortic wall remodeling in dissections occurring both in inherited conditions associated with aortic disease and in atherosclerosis (Noemi Cifani et al., "Stanford-A Acute Aortic Dissection, Inflammation, and Metalloproteinases: A Review," Annals of Medicine 47, no. 6 (2015): 441-46). Inflammatory cells contribute to aortic disease onset and progression (Zhen Yuan et al., "Abdominal Aortic Aneurysm: Roles of Inflammatory Cells," Frontiers in Immunology 11 (February 3, 2021): 609161). A multitude of anti-inflammatory agents have been evaluated in preclinical models of aortic disease (Georgia Skotsimara et al., "Aortic Wall Inflammation in the Pathogenesis, Diagnosis and Treatment of Aortic Aneurysms," Inflammation 45, no. 3 (June 2022): 965-76) and in man (B. Timothy Baxter et al., "Effect of Doxycycline on Aneurysm Growth Among Patients With Small Infrarenal Abdominal Aortic Aneurysms: A Randomized Clinical Trial," JAMA 323, no. 20 (May 26, 2020): 2029-38). Elevation of BHB has anti-inflammatory activities that may, in addition to its senolytic and reprogramming activities, contribute to preventing and healing aortic disease (Youm et al., "The Ketone Metabolite β-Hydroxybutyrate Blocks NLRP3 Inflammasome-Mediated Inflammatory Disease"; Markus Wortmann et al., "Inflammasomes in the Pathophysiology of Aortic Disease," Cells 10, no. 9 (September 15, 2021): 2433)⁴³. There is one recent report of an SGL2 inhibitor, originally developed to treat type two diabetes, showing a benefit in reducing aortic aneurysmal progression (Ortega et al., "SGLT-2 (Sodium-Glucose Cotransporter 2) Inhibition Reduces Ang II (Angiotensin II)-Induced Dissecting Abdominal Aortic Aneurysm in ApoE (Apolipoprotein E) Knockout Mice). This benefit was attributed to anti-inflammatory activity. Because anti-inflammatory activity of several agents was demonstrated to succeed in animal models of AAA yet has repeatedly failed to translate to man (Baxter Bt et al., "Non-Invasive Treatment of Abdominal Aortic Aneurysm Clinical Trial (N-TA(3)CT): Design of a Phase IIb, Placebo-Controlled, Double-Blind, Randomized Clinical Trial of Doxycycline for the Reduction of Growth of Small Abdominal Aortic Aneurysm," Contemporary Clinical Trials 48 (May 2016)) there can be no expectation, based on this report, that SGLT2 inhibition would be a successful strategy to treat AAA in man.

### SUMMARY OF THE INVENTION

Described herein are compositions and their use in methods for treating (a subject) with a vessel aneurysm or a dissection; or at risk for developing a vessel aneurysm or dissection or experiencing a rupture in a vessel. The vessel may be the aorta or other artery or vessel. The use in the treatment can comprise administering (to the subject an effective amount of) beta-hydroxybutyrate (BHB) or an SGLT2 and/or inhibitor or a combination of both. The compositions described herein are useful in maintaining the health and normal function of cells, that may make up vessels walls and/or repairing damage to vessels caused by genetic predispositions, environmental factors, and behaviour such smoking, (other risk factors such as) hypertension and advanced age and molecular, inflammatory epigenetic and/or (other processes which may) promote the development of aneurysm, dissection, or rupture. Other systems, methods, features, and advantages of the present disclosure will be or become apparent to one with skill in the art upon examination of the following drawings and detailed description. It is intended that all such additional systems, methods, features, and advantages be included within this description, be within the scope of the present disclosure, and be protected by the accompanying claims. In addition, all optional and preferred features and modifications of the described embodiments are usable in all aspects of the disclosure taught herein. Furthermore, the individual features of the dependent claims, as well as all optional and preferred features and modifications of the described embodiments are combinable and interchangeable with one another.

### BRIEF DESCRIPTION OF THE DRAWINGS

Aspects of the inventions are exemplified with reference to the following figures depicting chemical structures.

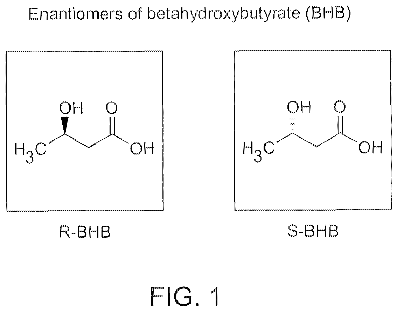

Figure 1 illustrates the two enantiomers of BHB. R-BHB is known to serve as a fuel molecule, to be involved in signaling and to play several roles in epigenetic modulation. S-BHB possess similar signaling and epigenetic activity; but in contrast to R-BHB is not a fuel molecule. If only signaling or epigenetic or both such activities are desired, S-BHB may be preferred over R-BHB due S-BHB being metabolized much more slowly. If, providing addition energy to tissue such as to the brain or heart is desirable, R-BHB may be preferred.

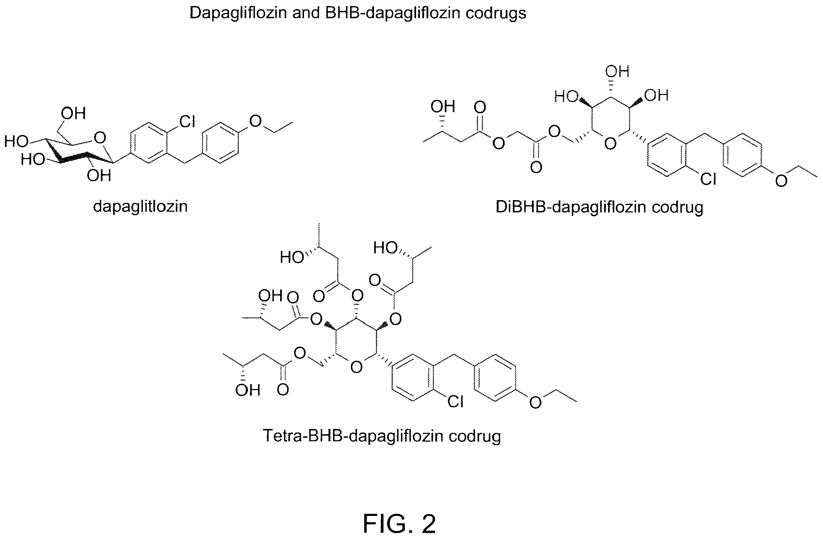

Figure 2 illustrates exemplary codrugs that may be used to deliver BHB and SGLT2i activity simultaneously. For purposes of illustration the SGLT2i activity is provided by dapagliflozin in Figure 2 (IUPAC name (2S,3R,4R,5S,6R)-2-{4-chloro-3-[(4-ethoxyphenyl)methyl]phenyl}-6-(hydroxymethyl)oxane-3,4,5-triol). Dapagliflozin is a C-glycosyl comprising β-D-glucose in which the anomeric hydroxy group is replaced by a 4-chloro-3-(4ethoxybenzyl)phenyl group. As shown in Figure 2, one or more BHB moieties may be linked to C-glycosyl. DiBHB dapagliflozin and TetraBHB dapagliflozin are depicted. One of ordinary skill in the art will appreciate that BHB activity delivered by such codrugs may be that of R-BHB or S-BHB and that other SGLT2 Inhibitors including bexaglifloxin, canagliflozin, empagliflozin, ertugliflozin, and luseoglifloizin may be linked to BHB using similar chemistry.

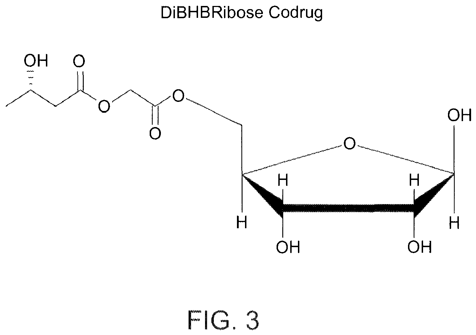

Figure 3 illustrates a codrug that may be used to deliver BHB and ribose activity simultaneously. Ribose activity and BHB activity may both improve heart function.

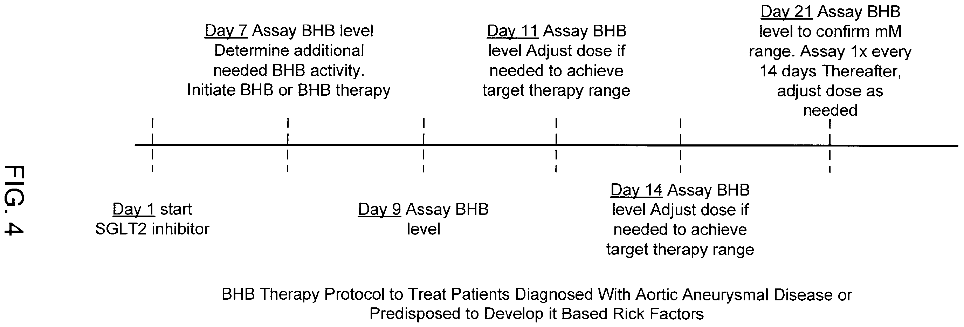

Figure 4 illustrates a BHB therapy protocol to treat patients diagnosed with aortic aneurysmal disease or predisposed to develop it based risk factors.

### SGLT2 INHIBITORS ALONE OR COMBINED WITH EXOGENOUS KETONES TO TREAT AORTIC ANEURYSMS, HEART FAILURE, CANCER, TO IMPROVE COGNITION AND FOR OTHER INDICATIONS

In healthy persons, serum levels of BHB varies and may be affected by diet, especially carbohydrate intake. BHB levels are often in the low micromolar range (-.15mM). Levels begin to rise after 12-16 h of fasting, reaching 1-2 mM after 2 days of fasting and 6-8 mM with prolonged starvation (Newman and Verdin, "β-Hydroxybutyrate," August 21, 2017; Nasser et al., "Effects of Ketogenic Diet and Ketone Bodies on the Cardiovascular System"; Mark Evans, Karl E. Cogan, and Brendan Egan, "Metabolism of Ketone Bodies during Exercise and Training: Physiological Basis for Exogenous Supplementation," The Journal of Physiology 595, no. 9 (May 1, 2017): 2857-71). The liver of a healthy adult is capable of producing up to 185 grams (g) of ketones (BHB + AcAc) per day during prolonged starvation. Normal production of ketones following an overnight fast in healthy adults is on the order of 30 to 60 grams per day. Studies indicate that even extremely elevated levels of BHB are safe acutely and that levels below ~6mM are likely safe for very long periods of time. Indeed, many now believe that BHB elevated in the range of 1-2mM chronically, imparts health benefits, including improved cognition, cardiovascular function, and reduced inflammation (Bendridi et al., "Ketone Bodies as Metabolites and Signaling Molecules at the Crossroad between Inflammation and Epigenetic Control of Cardiometabolic Disorders"; Nasser et al., "Effects of Ketogenic Diet and Ketone Bodies on the Cardiovascular System). It is further well appreciated that trying to elevate BHB by reliance on a ketogenic diet (KD) is difficult for most people. Also, the components of ketogenic diets may not be optimal from a nutrition and health perspective for many people. The classic KD is characterized by a 4:1 ratio of fats to carbohydrates and proteins, where for every 4 grams of fats, there is 1 gram of carbohydrates and proteins combined. Various modifications of the classic KD are currently in use. It is assumed that while ingesting a KD about 70%-80% of energy should come from fat, and the rest of the requirement should be derived from proteins and carbohydrate energy sources. The amount of carbohydrates should typically remain below ~ 50 grams/day.

Exogenous ketones typically supplements, may be ingested to raise BHB levels. Examples of exogenous ketone compounds include medium chain triglycerides, ketone salts and ketone esters. Many variants of these compounds have been studied for their ability to induce ketosis defined as >.5mM BHB, and for their impact on a range of outcomes, including physical performance (Poffe C, et al. Bicarbonate unlocks the ergogenic action of ketone monoester intake in endurance exercise. Med Sci Sports Exerc. 2021;53(2):431-41), (Cox PJ, Kirk T, Ashmore T, Willerton K, Evans R, Smith A, Murray AJ, Stubbs B, West J, McLure SW, et al. Nutritional ketosis alters fuel preference and thereby endurance performance in athletes. Cell Metab. 2016;24(2):256-68. doi:10.1016/j. cmet.2016.07.010)), cognitive function ( Waldman HS, Basham SA, Price FG, Smith JW, Chander H, Knight AC, Krings BM, McAllister MJ. Exogenous ketone salts do not improve cognitive responses after a high-intensity exercise protocol in healthy college-aged males. Appl Physiol Nutr Metab. 2018;43(7):711-7. doi:10.1139/apnm-2017-0724. Mujica-Parodi LR, Amgalan A, Sultan SF, Antal B, Sun X, Skiena S, Lithen A, Adra N, Ratai E-M, Weistuch C, et al. Diet modulates brain network stability, a biomarker for brain aging, in young adults. Proc Natl Acad Sci USA. 2020;117(11):6170-7.doi:10.1073/pnas.1913042117. Evans M, Egan B. Intermittent running and cognitive performance after ketone ester ingestion. Med Sci Sports Exerc.2018;50(11):2330-8. doi:10.1249/MSS.0000000000001700. Avgerinos KI, Egan JM, Mattson MP, Kapogiannis D. MediumChain Triglycerides induce mild ketosis and may improve cognition in Alzheimer's disease. A systematic review and meta-analysis of human studies. Ageing Res Rev. 2020;58:101001) blood glucose control and appetite regulation. First generation BHB supplements are ketone salts. These are marketed under many labels, such as Keto Sports, Keto Science, H.V.M.D. and many others. These products are often racemic mixtures of R-BHB and S-BHB salts, or mixtures of such salts enriched in R-BHB. A key reason that these products may be enriched with R-BHB is that most are marketed to improve energy, mental alertness, stamina, and exercise performance by giving the body an alternative energy source. Renowned BHB scientist, Dr Richard Veech, working with colleagues at Oxford University, developed a ketone ester supplement product. The ester form has the advantage of being free of salts and delivers two molecules of R-BHB per ketone ester. It is marketed as deltaG ketones (50 https://www.deltagketones.com/). Recently, a company called Juvenescence introduced the ketone ester bis-hexonly (R) -1-3 butanediol to elevate BHB (Journal of the American College of Nutrition https://doi.org/10.1080/07315724.2021.2015476). Ketone esters are of particular translational interest because they elevate blood ketone concentrations without an accompanying acid or mineral load (Soto-Mota A, Norwitz NG, Clarke K. Why a d-β-hydroxybutyratemonoester? Biochem Soc Trans. 2020;48(1):51-9. doi:10.1042/ BST20190240).

Many factors, including the nature of BHB and the understanding of its biology are favorable in the context of developing BHB as a pharmaceutical. These include: (i) BHB's nature as a endogenous metabolite that occurs at high elevation naturally (safety); (ii) decades of study of BHB's role in energy metabolism; (iii) experience inducing ketosis through diet to achieve desired changes (weight loss, enhanced athletic performance) and reduction in symptoms caused by disease i.e. epilepsy by a ketogenic diet (iv) the advent of supplements ketone salts (KS) and ketone esters (KE) which have made it easier to induce ketosis and motivated useful studies of BHB Pk and side effect profile; and (v) favorable inherent drug-like characteristics, including high solubility and oral bioavailability.

However, there is a significant barrier that needs to be overcome in order to enable BHB therapy to be achieved through a pharmaceutical approach. There is currently no means to provide for the sustained elevation of BHB required to achieve concentrations that may be needed for most people to realize therapeutic benefit (~1.25mM to 2.0mM) in a manner that facilitates compliance. This invention provides such means.

Numerous studies reporting the pharmacokinetics of BHB supplements and their general safety have been reported in the literature (Stubbs et al., "On the Metabolism of Exogenous Ketones in Humans"; Bernard Cuenoud et al., "Metabolism of Exogenous D-Beta-Hydroxybutyrate, an Energy Substrate Avidly Consumed by the Heart and Kidney," Frontiers in Nutrition 7 (2020): 13, https://doi.org/10.3389/fnut.2020.00013; Vittal Shivva et al., "The Population Pharmacokinetics of D-β-Hydroxybutyrate Following Administration of (R)-3-Hydroxybutyl (R)-3-Hydroxybutyrate," The AAPS Journal 18, no. 3 (May 2016): 678-88). The upshot of these is that: (i) the naturally occurring enantiomer of (D/R) BHB is very rapidly metabolized; (ii) (S/L) BHB is sustained much longer (more than 2x) than (D/R) BHB in plasma (iii) large quantities of BHB must be consumed in order to elevate BHB mM into a therapeutic range and keep it there, particularly if R-BHB is ingested for this purpose. Soto-Mota and colleagues reported the results of a 28-day study of KE-BHB involving 28 volunteers. KE releases only R-BHB, not S-BHB. The study examined the ability of a KE R-BHB (ketone ester) supplement to support ketosis induction and, also, how well elevated R-BHB was tolerated. The maximum BHB plasma concentration reached in each participant ranged from 1.8 to 6.3 mM, with the average maximum range being 4.1 ± 1.1 mM. As expected, sustained elevation of BHB was safe based on all measured parameters. Unlike a ketogenic diet, ingesting the KE supplement had no effect on body weights or composition, fasting blood glucose, cholesterol, triglyceride, or electrolyte concentrations, nor blood gases or kidney function. Volunteers drank 25 ml (containing 26.8 g) of KE R(D)-BHB three times per day comprising 80.5 grams of ketone ester. A more sophisticated modeling of the population pharmacokinetics of KE R- BHB (single dose) was published by Shivva et.al. Healthy volunteers (n=37) were given a single drink of the ketone monoester, following which 833 blood BHB concentrations were measured. Two formulations and five different doses of ketone monoester were used. Their data makes it crystal clear that elevated plasma D-BHB is not sustained, that high doses are needed to achieve ketosis, and that formulation ingredients (dairy fat milkshake v citrus drink) can significantly influence plasma levels. The safety and Pk associated with a more recently introduced BHB supplement di-ester, bishexanoyl (R)-1,3-butanediol (BH-BD) has been studied and reported ((Christopher D. Crabtree et al., "Bis Hexanoyl (R)-1,3-Butanediol, a Novel Ketogenic Ester, Acutely Increases Circulating r- and s-β-Hydroxybutyrate Concentrations in Healthy Adults," Journal of the American Nutrition Association, March 25, 2022), (Brianna J. Stubbs et al., "In Vitro Stability and in Vivo Pharmacokinetics of the Novel Ketogenic Ester, Bis Hexanoyl (R)-1,3-Butanediol," Food and Chemical Toxicology: An International Journal Published for the British Industrial Biological Research Association 147 (January 2021): 111859), (Brianna J. Stubbs et al., "Toxicological Evaluation of the Ketogenic Ester Bis Hexanoyl (R)-1,3-Butanediol: Subchronic Toxicity in Sprague Dawley Rats," Food and Chemical Toxicology: An International Journal Published for the British Industrial Biological Research Association 150 (April 2021): 112084), (Kristin M. Nieman, Joshua C. Anthony, and Brianna J. Stubbs, "A Novel Powder Formulation of the Ketone Ester, Bis Hexanoyl (R)-1,3-Butanediol, Rapidly Increases Circulating β-Hydroxybutyrate Concentrations in Healthy Adults," Journal of the American Nutrition Association, October 24, 2022, 1-8)). A dose range needed to elevate and maintain therapeutic levels of R-BHB is feasible through the use of for example, sprinkle formulation which may also incorporate ingredients that allow for buffering if needed and for extended release if desirable. Sixty-five sprinkle formulated products have been FDA approved (Han Sol Lee et al., "Sprinkle Formulations-A Review of Commercially Available Products," Asian Journal of Pharmaceutical Sciences 15, no. 3 (May 2020): 292-310). An example of a gram quantity dose sprinkle is uridine triacetate, a pyrimidine analog and marked as Vistogard or Xuridin. It was approved in 2015 for persons with genetic diseases that impair the function of uridine monophosphate synthetase and for certain chemotherapy toxicity.

### R-BHB AND S-BHB POSSESS DIFFERENT ACTIVITIES SUITABLE TO IMPART THERAPEUTIC BENEFIT

For some indications and purposes elevated "R-BHB activity" alone may be adequate or preferred. For others, needed elevated "BHB activity" be achieved by the presence of both R-BHB and S-BHB. It is widely reported that S-BHB is sustained much longer in plasma than R-BHB. Stubbs et.al, (Stubbs et al., "On the Metabolism of Exogenous Ketones in Humans) provides very useful Pk data pertaining to S-BHB. S-BHB possesses desirable plasma stability and through signaling and epigenetic activities, offers therapeutic benefits. It appears that R-BHB and S-BHB play similar, but perhaps not identical roles, in signaling and epigenetics but that only R-BHB serves as a fuel molecule. Delivery of exogenous BHB, R-BHB in particular, is known to improve cardiac function in preclinical models. A trial is underway to explore the potential of exogenous R-BHB in metabolic disease and cognition (NCT04421014). The addition of ketone bodies to working rat hearts perfused with glucose significantly increases cardiac output and efficiency in a manner similar to insulin (Keon CA, Tuschiya N, Kashiwaya Y, Sato K, Clarke K, Radda GK, Veech RL. Substrate dependence of the mitochondrial energy status in the isolated working rat heart. Biochem Soc Trans. 1995;23:307S. [PubMed: 7672336]). A body of literature underscores an emerging appreciation that as the failing human heart progressively loses its capacity to oxidize glucose, ketone bodies provide a potential alternative substrate, since ketone oxidation bypasses the complex dysregulation of the beta-oxidation pathway and pyruvate dehydrogenase complex in heart failure (Circulation. 2020 June 02; 141(22): 1800-1812. doi:10.1161/CIRCULATIONAHA.119.045033). One study demonstrated increase in heart rate, pulse pressure (mediated by lowering of diastolic blood pressure), and a 75% increase myocardial blood flow, and near halving of glucose uptake by elevating BHB. The mechanism of the increased heart rate is not clear but may be a compensatory response to vasodilation rather than a sympathetic response, which is typically downregulated by ketones (Circulation. 2020 June 02; 141(22): 1800-1812. doi:10.1161/CIRCULATIONAHA.119.045033). Ketone salts have been studied for the purpose inducing of ketosis and been found to be safe ((Tobias Fischer et al., "Effect of a Sodium and Calcium DL-β-Hydroxybutyrate Salt in Healthy Adults," Journal of Nutrition and Metabolism 2018 (2018): 9812806), (Hunter S. Waldman et al., "Exogenous Ketone Salts Do Not Improve Cognitive Responses after a High-Intensity Exercise Protocol in Healthy College-Aged Males," Applied Physiology, Nutrition, and Metabolism = Physiologie Appliquee, Nutrition Et Metabolisme 43, no. 7 (July 2018): 711-17), (Elena Gross et al., "Efficacy and Safety of Exogenous Ketone Bodies for Preventive Treatment of Migraine: A Study Protocol for a Single-Centred, Randomised, Placebo-Controlled, Double-Blind Crossover Trial," Trials 20, no. 1 (January 17, 2019)). Likewise ketone esters are safe and have no untoward cardiovascular effects after six weeks of use (https://doi.org/10.1186/s13063-018-3120-7). KE produces a rapid rise of blood levels of BHB in humans to nearly 3 mmol/L within 60 minutes of consumption of 282 mg/kg of KE (Clarke K, Tchabanenko K, Pawlosky R, Carter E, Todd King M, Musa-Veloso K, Ho M, Roberts A, Robertson J, Vanitallie TB, Veech RL. Kinetics, safety and tolerability of (r)-3-hydroxybutyl (r)-3-hydroxybutyrate in healthy adult subjects. Regul Toxicol Pharmacol. 2012;63:401-408. [PubMed: 22561291]). Early studies indicate that athletes, in particular, appear to undergo metabolic reprogramming that result in favorable ketone utilization during exercise. In a recent blinded, crossover study of endurance athletes, ketone ester therapy increased exercise distance by an average of 411 meters over 30 minutes cycling duration, demonstrating that ketone therapy improves exercise capacity among highly trained athletes, though a ceiling effect may be reached in that population. BHB therapy, especially with R-BHB, may hold great benefit in pathologic conditions that cause metabolic dysregulation and where incremental improvements in energy transduction may translate to increases in exercise capacity, such as in heart failure (Hunter WG, Kelly JP, McGarrah RW 3rd, Khouri MG, Craig D, Haynes C, Ilkayeva O, Stevens RD, Bain JR, Muehlbauer MJ, Newgard CB, Felker GM, Hernandez AF, Velazquez EJ, Kraus WE, Shah SH. Metabolomic profiling identifies novel circulating biomarkers of mitochondrial dysfunction differentially elevated in heart failure with preserved versus reduced ejection fraction: Evidence for shared metabolic impairments in clinical heart failure. J Am Heart Assoc. 2016;5:e003190. [PubMed: 27473038).

One central innovation provided by this invention is that it is, to the best of our knowledge, the first identification of an indication for which elevated S-BHB alone has therapeutic benefit, specifically, the ability to maintain and to heal or replace damaged vascular smooth muscle cells, thereby reducing the risk of aneurysm, dissection or rupture and arresting or reversing aneurysm.

### DETAILS OF THE INVENTION(S)

There is an unmet need for a means (which the invention provides) to achieve BHB therapy via a pharmaceutical approach to treat several diseases, including, but not limited to, aortic aneurysmal disease and reduce risk for dissection or rupture, heart failure, cognition, and epilepsy. This need arises because currently available materials that elevate BHB do not support sustained therapeutic levels of BHB. Some are formulated with salts that are contra-indicated for many persons, many are extremely unpalatable, may cause gastrointestinal discomfort and overall are incompatible with compliance.

As disclosed in PCT/US2022/073582 certain coinventors on this application first showed that very unexpectedly, elevating either R-BHB or S-BHB is beneficial to treat aortic aneurysmal disease.

The inventors of the present invention recognize that in order to treat aortic aneurysmal and other human diseases optimally, it is necessary to provide novel compositions that achieve elevated BHB activity in a safe and effective manner. For some conditions, elevation of R-BHB only may be preferred. For others, elevating both R-BHB and S-BHB may be preferred. The compositions of this invention can comprise: SGLT2 inhibitor-BHB or ribose-BHB codrugs; and (ii) SGLT2 inhibitors in combination with R-BHB or S-BHB, as free acids, or as salts or esters. SGLT2 inhibitors can elevate predominantly R-BHB only. SGLT2 inhibitor-BHB codrugs may deliver SGLT2i and enhanced R-BHB activity or SGLT2i and enhanced R-BHB and S-BHB activity or SGLT2i and additional S-BHB activity. Ribose-BHB codrugs may deliver ribose and R-BHB activity or ribose and both R-BHB and S-BHB or ribose and S-BHB only activity. Various embodiments contemplated herein may include, but need not be limited to, one or more of the following:

### EMBODIMENT 1: SGLT2I AND ENHANCED BHBACTIVITY

An SGLT2 inhibitor may be taken from the group comprised of, dapagliflozin, canagliflozin, empagliflozin, ertugliflozin, ipragliflozin, luseogliflozin or tofogliflozin. A drug able to deliver BHB activity may be a BHB acid or BHB salt or ketone ester. BHB acids, BHB salts or ketone esters may be prepared to deliver R-BHB activity only, R-BHB and SBHB activity or S-BHB activity only. The enantiomers of BHB are shown in Figure 1. A ketone ester may be prepared in a variety of ways such as, wherein the BHB ester is in the S-BHB-S-1.3 butanediol configuration, the R-BHB-R-1.3 butanediol configuration, the S-BHB-R-1.3 butanediol configuration, or R-BHB-S-1.3 butanediol.

An SGLT2 inhibitor and BHB acid or BHB salt or ketone ester may be administered simultaneously or at different times during the day to a patient in need of SGLT2i and enhanced BHB activity at doses needed to achieve plasma exposures deemed safe and effective. Pharmacokinetic parameters of various SGLT2 inhibitors are known (Table 1).

**Table 1: Pharmacokinetic Parameters of Various SGLT2-Inhibitors**

| Name of drug | Bioavailability | Protein binding | tₘₐₓ (hours) | t_{1/2} (hours) | Cₘₐₓ | SGLT₂ selectivity over SGLT₁ |
|---|---|---|---|---|---|---|
| Canagliflozin | 65% (300 mg dose) | 99% | 1-2 | 10.6 (100 mg dose); | 1096 ng/mL (100 mg dose); | 250 fold |
| | | | | 13.1 (300 mg dose) | 3480 ng/mL (300 mg dose) | |
| Dapagliflozin | 78% | 91% | 1-1.5 | 12.9 | 79.6 ng/mL (5 mg dose); | 1200 fold |
| | | | | | 165.0 ng/mL (10 mg dose) | |
| Empagliflozin | 90-97% (mice); 89% (dogs); 31% (rats) | 86.20% | 1.5 | 13.2 (10 mg dose); | 259nmol/L (10 mg dose); | 2500 fold |
| | | | | 13.3h (25 mg dose) | 687nmol/L (25 mg dose) | |
| Etrugliflozin | 70-90% | 95% | 0.5-1.5 | 11-17 | 268 ng/mL (15 mg dose) | 2000 fold |
| Ipragliflozin (50 mg) | 90% | 96.30% | 1 | 15-16 (50 mg dose) | 975 ng/mL | 360 fold |
| Luseogliflozi n | 35.3% (male rats); 58.2% (female rats); 97.2% (male dogs) | 96.0-96.3% | 0.625±0.354 | 9.24±0.928 | 119±27.0 ng/mL | 1650 fold |
| Tofogliflozin (10 mg) | 97.50% | 83% | 0.75 | 6.8 | 489 ng/mL | 2900 fold |

| | | | | | | |
|---|---|---|---|---|---|---|
| • Cₘₐₓ. Maximum serum concentration that drug achieves in body after the drug has been and administered • tₘₐₓ: Time to achieve maximum plasma concentration • t_{1/2}: Biological half-life | | | | | | |

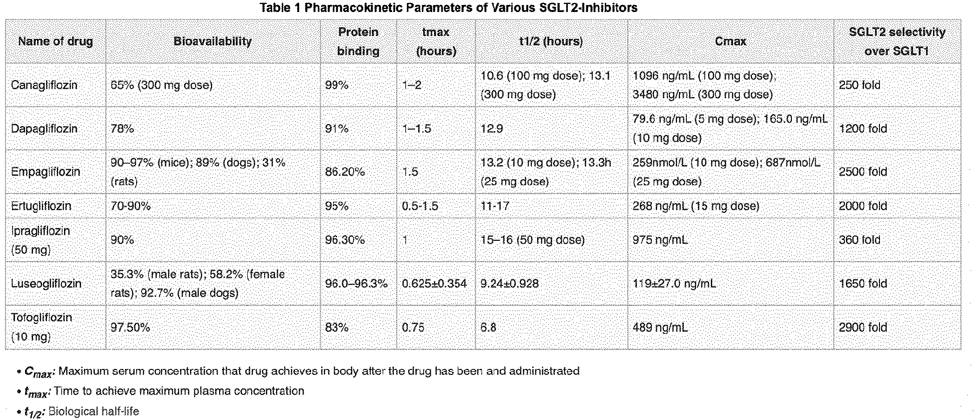

Actives capable of delivering both SGLT2i and additional BHB activity may be formulated together in sprinkles, capsules or other pharmaceutical forms that may possess extended release properties.⁶⁴

### EMBODIMENT 2: RIBOSE AND BHB ACTIVITY

Heart failure with preserved ejection fraction (HFpEF), also called diastolic HF, is a debilitating cardiovascular disease. Researchers are investigating mitochondrial dysfunction and impaired cardiac bioenergetics as a possible pathophysiologic mechanism behind the pathogenesis of HFpEF. The administration of ribose may improve heart function (Janet D. Pierce et al., "Effects of Ubiquinol and/or D-Ribose in Patients With Heart Failure With Preserved Ejection Fraction," The American Journal of Cardiology 176 (August 1, 2022): 79-88). The administration of BHB may also improve heart function. As shown in Figure 3 this invention provides a novel codrug that allows for convenient administration of ribose and BHB activity simultaneously in the same sprinkle, capsule, or other formulation.

The (embodiments and) formulations of this invention may be administered (via a modality selected from the group consisting of) intra-peritoneal administration, IV infused administration, topical administration, oral administration, inhalation administration, transdermal administration, and/or subdermal depot administration.

Such formulations may comprise tablets, powder, granules, immediate-release (IR) capsules, including extended-release (ER) capsules, delayed-release (DR) capsules, and/or multiarticulate drug delivery system (MDDS) (which may allow for more even levels of an active (here BHB) and a reduced frequency of dosing).

The (embodiments and) formulations of this invention may (be in admixture with) a carrier or excipient, suitably comprising a sugar, starch, cellulose, powdered tragacanth, malt, gelatin, talc, cocoa butter, suppository wax, oil, mineral oil, food coloring, natural flavorings, artificial flavorings, citric acid, glycol, polyol, ester, agar, buffering agent, alginic acid, isotonic saline, Ringer's solution, ethyl alcohol, polyester, polycarbonate polyethylene glycol, polyvinylpyrrolidone, water, polyanhydride, and/or magnesium stearate. The composition may be formulated for oral administration.

### DETAILED DESCRIPTION OF USE OF SLGT2 INHIBITORS CODRUGS AND BHB PRODRUGS INCLUDING DOSING IN ACCORDANCE WITH THE INVENTION(S).

The embodiments or formulations described herein may be dosed to achieve the desired SGHLT2i, BHB and/or ribose activity. The level of desired BHB activity can be reflected in a target mM BHB (plasma concentration) over a unit of time. Preferred desired BHB concentrations may be in the ranges of 0.25-0.75mM, 0.75mm to 1.25mM, 1.25 to 1.75mM, 1.75mM-2.25mM or over 2.25mM. For many indications a preferred dose of BHB will be in a range between 1.0mM and 2mM. Doses that achieve 2.25mM+ may be indicated for certain conditions, or for certain periods of therapy. Desired levels may be maintained for 4 hours, 8 hours or 12+ hours a day. Before determining a BHB dose for any patient, the patient's resting BHB mM level should be confirmed since this is known to vary. In addition, when providing SGLT2i and BHB activity is the goal, BHB mM level of a given patient should be ascertained after they have been talking an SGLT2 inhibitor long enough to reach a steady state concentration of BHB resulting solely from SGLT2i activity (without any direct BHB activity) , generally about one week. This may be done using standard analytical tests such as mass spectrometry. It may also be done at home or in a doctors' office using commercially available, single drop of blood on a test strip monitor devices, to detect R-BHB. An exemplary product is the Keto-Mojo Blood Glucose and Ketone home monitor ( https://keto-mojo.com/). SGLT2 inhibitors may be dosed as currently prescribed. The doses for FDA approved SGLT2 inhibitors are known. SGLT2 inhibitors typically elevate BHB plasma concentration about 2-4-fold. Elevation is often greater in a patient with diabetes, but some elevation commonly occurs in nondiabetic persons as well. The SGLT2 inhibitor may be taken as a tablet or capsule or (if administered using BHB esters, salts, prodrugs or if SGLT2 inhibitor BHB codrugs are given), mixed into a sprinkle formulation or capsule or tablet.

For the majority of patients, the post SGLT2 inhibitor level of BHB mM will be in the range of 0.2 to 0.4 mM. This value should be verified using analytical testing (mass spectrometry) or with a home meter or monitor. Sufficiency of SHLT2i and BHB activity may be assessed for different indications in different ways known to physicians and other care takers. If more R-BHB or S-BHB activity is desired dosing in particular of BHB may be increased. An SGLT2 inhibitor - BHB codrug may be beneficial to increase R-BHB activity or to add S-BHB activity. S-BHB activity would be preferred if the indication is to treat aortic disease therapy. Again, the BHB mM levels achieved following administration of a codrug should be confirmed. Patients and care givers should be aware that ketone strip test meters may not detect S-BHB content (Prins et al. Nutr Metab (Lond) (2020) 17:81). In all cases, an SGLT2 inhibitor alone is expected to elevate R-BHB but not, S-BHB appreciably. The additional needed BHB activity in terms of mM of BHB may be determined simply by subtracting the post SGLT2i BHB mM value from the target therapeutic value (TTV). As an example, if the desired TTV is in the ranges of 1.0-1.5mM BHB for 12+ hours a day and the post SGLT2i BHB mM value is ~0.3mM for 12+ hours a day, the additional needed BHB activity expressed as concentration is ~1.0mM BHB for 12+ hours a day. It is reported that continuous BHB monitors are in development by companies such as Abbott labs. These should be used where available.

Adding BHB activity may be done by administering (to the patient) the amount of the second agent (BHB or a BHB prodrug) required to achieve the desired BHB activity. It is appreciated that the pharmacokinetic response to exogenous BHB is variable and complex. One study of the response to ingestion of a ketone ester drug that delivers two R-BHBs per ester molecule, determined that covariates impacting BHB pharmacokinetics include formulation (on relative oral bioavailable fraction and absorption rate constant) and dose (on relative oral bioavailable fraction). Lean body weight (on first-order clearance) and sex (on apparent volume of distribution). In general, the absorption and elimination of BHB is not fully understood and the dose response (concentration level) is not linear (Shivva et al., "The Population Pharmacokinetics of D-β-Hydroxybutyrate Following Administration of (R)-3-Hydroxybutyl (R)-3-Hydroxybutyrate.") Hence, it is strongly preferred in the practice of this invention to tailor dosing for each patient and to monitor BHB levels periodically during the course of therapy to allow for dose adjustment. Literature data, and routine, known methods for tracking levels of therapies may be used to arrive upon the optimal dose of to achieve the desired BHB activity. In general, dosing will be more frequent if the additional BHB or BHB prodrugs delivers R-BHB only in contrast to use of an additional BHB or BHB prodrug that delivers S-BHB or both R-BHB and S-BHB.

### RECOMMENDED DOSING TO TREAT OR PREVENT AORTIC DISEASE

If the goal is to provide a safe and effective therapy to treat extant abdominal or thoracic aneurysm, dissection or rupture or to provide prophylactic protection to persons at risk to develop any of these, the preferred practice of this invention would be to provide for safe and effective SGLT2i activity including the maintenance of autophagy and elevation of Sirtuin proteins and safe and effective BHB anti-inflammatory signaling activity and epigenetic modulation that protects endothelial cells and vascular smooth muscle cells (vSMC) and promotes the maintenance of functional vSMC phenotypes. In accordance with this invention this would be accomplished by treating (the patient or person at risk) with an SGLT2 Inhibitor at the dose recommended to treat heart failure patients with that SGLT2 inhibitor; combined with a sufficient dose of S-BHB to support elevation of BHB to about ~1.25mM-2.0mM, suitably for ten (10)+ hours a day.

The inventors contracted to have a physiologically based pharmacokinetic modeling done for BHB based on what is has been published about ketone ester and BHB salt pharmacokinetics and what is known about esterase breakdown of ketone ester, absorption of BHB and transport of BHB and other parameters. The conclusion is that about 5-6 grams of ketone ester of the S-BHB-S-1.3 butanediol configuration would be an optimal dose (to elevate BHB to therapeutic levels to treat AAA or TAA).

### ADDITIONAL BHB ACTIVITY: FORMULATION TYPES

R-BHB activity formulation (R-BHB AF) means a ketone salt that releases R-BHB only or ketone ester that releases 2 R-BHB per molecule of KE. If the KE is formulated to offer extended release it shall be termed R-BHB AFEx. Racemic-BHB formulation (Rac-BHB AF) shall mean a racemic ketone salt or ketone ester that releases 1 R-BHB and 1 S-BHB per molecule. If KE is formulated to offer extended release it shall be termed Rac-BHB AFEx. SBHB activity formulation (S-BHB AF) shall mean a ketone salt that releases only S-BHB or a ketone ester that releases 2 S-BHBs per molecule. If the KE is formulated to offer extended release it shall be termed S-BHBAFEx

### DOSE SPRINKLE FORMULATION PACKETS MAY CONTAIN ANY FORMULATION TYPE

High Dose (HD) 24 grams of KE
Medium (MD) 12 grams of KE
Low Dose (LD) 2 grams of KE (may be in capsule form)

**Table 2. Recommended Baseline Dosing of BHB or BHB Prodrug to Achieve Therapeutic Levels**

| Indication-Purpose of Therapy | Therapeutic Range | Duration Endpoint | Formulation Type | Packets/Schedule |
|---|---|---|---|---|
| Treat Established Small AA or Post Dissection TAA with SGLTi2 and R-BHB activity (not S-BHB) for SSGLBHB see [0035] and [0036] above. | 1.0mM-1.5mM | Until expansion arrested or reversed + 6 months (if small AA) | R-BHB-AFEx | HD morning MD morning+5hrs HD before bed |
| | | Ongoing if to treat post dissection TAA) | | |
| Once an endpoint is achieved, periodic imaging will be needed to determine if continued therapy is appropriate | | | | |
| High Risk for TAA or TAAD | 1.0mM-1.5mM | | | MD morning HD + 6 hours |
| Epilepsy | 1.5mM-2.0mM | | R-BHB-AFEx | HD morning |
| | | | | MD morning+5hrs HD before bed (adjust with R-BHB AF only) |
| Heart Failure | 1.5mM-2.0mM | | R-BHB-AFEx | HD morning |
| | | | | MD morning+5hrs HD before bed (adjust with R-BHB AF only) |
| Cancer (blood or solid tumor) | 1.5mM-2.0mM | ongoing | S-BHB-AF or Rac-BHB-AFEr | HD morning |
| | | | | MD morning+5hrs HD before bed |

It is also within the scope of this invention to increase dosing to higher therapy by increments of 0.5mM such as 1.5mM to 2.0mM, 2.0mM to 2. 5mM and so on up until 5.0mM if beneficial for a particular patient by adding dose packets.

The steps to be taken during the first 21 days of therapy to ascertain the correct number of packets and schedule to be used for each patient to address their need for BHB therapy are summarized in the below SGLT2i BHB Activity Therapy Protocol figure. In general, if dosing is resulting in BHB mM values below the desired therapeutic range low dose packets should be added. Each low dose packet should elevate mM BHB -0.2-0.3. If the low dose packet is R-BHB the elevation should be expected to peak within 1 hour of ingestion and persist for ~2.5 hours. If the low dose packet is S-BHB the elevation should be expected to peak within 1 hour of ingestion and persist for -4-5 hours. If dosing is resulting in BHB mM values above the desired therapeutic range then one or more packets should be exchanged for one or packets that provide fewer grams.

### SYNTHESES AND FORMULATIONS

There is a voluminous body of scientific and patent literature that details methods of syntheses of myriad SGLT2 inhibitors, including those exemplified in the embodiments of this invention. A few are referenced here ((Jyotsana Pandey and Akhilesh K. Tamrakar, "SGLT2 Inhibitors for the Treatment of Diabetes: A Patent Review (2013-2018)," Expert Opinion on Therapeutic Patents 29, no. 5 (May 2019): 369-84), (Kwang-Seop Song et al., "Synthesis and SAR of Thiazolylmethylphenyl Glucoside as Novel C-Aryl Glucoside SGLT2 Inhibitors," ACS Medicinal Chemistry Letters 2, no. 2 (February 10, 2011): 182- 87)). Likewise, there is a voluminous body of scientific and patent literature that details methods of syntheses that detail strategies for making BHB, BHB salts, BHB esters prodrugs that would apply directly to preparing codrugs in accordance with this invention. A few are referenced here. ((Musa Ahmed et al., "Ester Prodrugs of Ketoprofen: Synthesis, In Vitro Stability, In Vivo Biological Evaluation and In Silico Comparative Docking Studies Against COX-1 and COX-2," Current Drug Discovery Technologies 13, no. 1 (2016): 41-57), (Kevin Beaumont et al., "Design of Ester Prodrugs to Enhance Oral Absorption of Poorly Permeable Compounds: Challenges to the Discovery Scientist," Current Drug Metabolism 4, no. 6 (December 2003): 461-85),(Christian Perez, Kevin B. Daniel, and Seth M. Cohen, "Evaluating Prodrug Strategies for Esterase-Triggered Release of Alcohols," ChemMedChem 8, no. 10 (October 2013): 1662-67)). Preparing compounds possessing SGLT2i and enhanced BHB activity as shown in Figure 2 or compounds having ribose and BHB activity as shown in Figure 3 is also within the ordinary skill of medicinal chemists (Perez, Daniel, and Cohen, "Evaluating Prodrug Strategies for Esterase-Triggered Release of Alcohols"; Beaumont et al., "Design of Ester Prodrugs to Enhance Oral Absorption of Poorly Permeable Compounds"). Many formulations suitable for use with ketones have been described in the literature pertaining to the use of ketone supplements and in patent filings. Two pertinent patent filings are Pub No US 2020/0222353 A1 and Patent No.: US 11,202,769 B2. Many commercial companies offer services in preparing sprinkle and extended-release formulations. These include Catalent Inc., Alcambi Inc. and Latitude Pharmaceuticals. Sixty-five sprinkle formulated products have been FDA approved (Han Sol Lee et al., "Sprinkle Formulations-A Review of Commercially Available Products," Asian Journal of Pharmaceutical Sciences 15, no. 3 (May 2020): 292-310). An example of a gram quantity dose sprinkle is uridine triacetate, a pyrimidine analog and marked as Vistogard or Xuridin. It was approved in 2015 for persons with genetic diseases that impair the function of uridine monophosphate synthetase and for certain chemotherapy toxicity.

## Claims

1. A composition comprising an SGLT2 inhibitor and beta hydroxybutyrate (BHB) or a prodrug thereof, preferably formulated for (simultaneous) oral delivery.

2. A composition according to claim 1 for use in treating aortic disease and/or heart failure and/or which is a pharmaceutical, medical or veterinary composition.

3. A composition according to claim 1 or 2 wherein the SGLT2 inhibitor comprises one or more of the following: dapagliflozin, canagliflozin, empagliflozin, ertugliflozin, ipragliflozin, luseogliflozin or tofogliflozin.

4. A composition according to any preceding claim wherein a prodrug of beta hydroxybutyrate (BHB) is used or present, suitably formulated for simultaneous oral delivery, wherein the BHB prodrug is a salt.

5. A composition according to any preceding claim wherein the BHB prodrug is ketone ester (KE).

6. A composition according to any preceding claim comprising an SGLT2 inhibitor and S- beta hydroxybutyrate (BHB) and/or which is formulated for (simultaneous) oral delivery.

7. A composition according to any preceding claim comprising an SGLT2 inhibitor and S-BHB-S-1,3 butanediol.

8. A composition according to any preceding claim comprising an SGLT2 inhibitor and S-BHB-S-1,3 butanediol and/or which is formulated for simultaneous oral delivery or extended release.

9. A composition according to any preceding claim comprising a codrug having SGLT2i and enhanced BHB activity as shown in Figure 2.

10. A composition according to any preceding claim for treating heart failure comprising a codrug having ribose and BHB activity as shown in Figure 3.

11. A composition according to any preceding claim for use in treating a vessel aneurysm or a dissection; or an individual at risk for developing a vessel aneurysm or dissection or experiencing a rupture in a vessel.

12. A product comprising an SGLT2 inhibitor and beta hydroxybutyrate (BHB) or a prodrug thereof for simultaneous, separate or sequential use in treating aortic disease and/or heart failure.

13. An SGLT2 inhibitor and beta hydroxybutyrate (BHB) or a prodrug thereof for use in medicine or in a medicament.

14. The use of a composition according to any of claims 1 to 11 use in treating aortic disease and/or heart failure.
